# EUROPEAN PATENT SPECIFICATION

(11) **EP 1 696 788 B1**
(45) Date of publication and mention of the grant of the patent: **17.08.2011**
(21) Application number: 04806684.9
(22) Date of filing: 22.12.2004
(51) Int. Cl.: A61B 1/04

(54) **DEVICE FOR IN-VIVO IMAGING OF A BODY LUMEN**
VORRICHTUNG FÜR DIE IN-VIVO-DARSTELLUNG EINES KÖRPERLUMENS
DISPOSITIF D'IMAGERIE IN VIVO D'UNE LUMIERE CORPORELLE

(30) Priority: 24.12.2003 US 531997 P
(43) Date of publication of application: 06.09.2006
(73) Proprietor: Given Imaging Ltd., 20692 Yoqneam (IL)
(72) Inventor: GAT, Daniel, 36863 Haifa (IL); GILAD, Zvika, 34987 Haifa (IL)
(74) Representative: Harrison Goddard Foote
(86) International application number: PCT/IL2004/001153
(87) International publication number: WO 2005/060348

(56) References cited:
- WO-A-2004/028335
- WO-A-2004/054430
- DE-A1- 10 028 155
- US-A- 5 833 603
- US-A- 5 984 875
- US-A1- 2003 018 280
- US-A1- 2003 023 150

## Description

### FIELD OF THE INVENTION

The present invention relates to the field of in vivo diagnostics. More specifically, the present invention relates to a device for imaging a body lumen.

### BACKGROUND OF THE INVENTION

Dyspeptic symptoms (e.g., dyspepsia) constitute a major reason for physician visits and referral for gastroenterology consultation. Some pathologies of the gastrointestinal (GI) tract involve for example epithelial damage, erosions, and ulcers. For example, inflammation of the GI tract mucosa (typically in the stomach), such as gastritis, can be characterized, inter alia, based on the endoscopic appearance of the gastric mucosa (e.g., varioliform gastritis). Other pathologies may involve irregularities or abnormal appearances of folds, polyps or color indications (such as bleeding) on the GI tract wall. Detection of these pathologies at an initial stage plays an important role in enhancing the probability of a cure.

Screening populations for initial signs of GI tract pathologies is typically carried out by non invasive methods including x-ray series in which a patient intakes x-ray opaque (e.g., radio-opaque) material (e.g., barium, gastrographine, or others). The material resides for some time on the walls of the GI tract, enabling examination of the x-ray images of the GI tract. This technique has several drawbacks, such as low detection rate and exposure to x-ray radiation. Other screening methods include viewing the GI tract walls or lumens by means of appropriate endoscopes. Risks associated with some endoscopy procedures include injury to the bowel wall, bleeding, and aspiration. Additionally, the endoscopy procedure is a cause of discomfort, pain and vomiting and may initiate fear in many patients. Such risks, along with the discomfort and fear, are often used as justifications by patients for delaying or altogether avoiding gastroscopic diagnosis.

Visualization of the GI tract, including the more difficult to reach areas, such as the small intestine, is possible today using an ingestible imaging device. Images of the GI tract are obtained by a miniature image sensor carried by the device and are transmitted to an external recorder to be later viewed on a workstation. Ingestible devices may be moved through the GI tract by the natural movement of peristalsis.

However, in voluminous lumens such as the stomach or large intestine, peristaltic movement may not be efficient in moving the device to cover the entire lumen.

It is known from DE 100 28 155 to provide a rod-shaped endoscope designed to be pushed into a patient. The endoscope includes inflatable balloons that serve to separate parts of the endoscope from tissue within a body lumen.

### SUMMARY OF THE INVENTION

There is thus provided, according to embodiments of the invention, a device for imaging a body lumen. According to an embodiment of the invention there is provided a device for imaging typically voluminous, usually liquid filled body lumens, for example, the stomach.

According to an embodiment of the invention a typically floatable imaging device, or a device having other suitable weight or mass distribution or specific gravity, is moved through a body lumen, such as the large intestine or stomach, by using the passage of a volume of liquid within and/or through the body lumen.

The device may be used in a method for imaging body lumens, such as the large intestine or the stomach. The method may comprise the step of inserting a floatable in vivo device into a patient lumen, for example by swallowing the imaging device, preferably after which a patient ingests a volume of liquid, for example water or juice. The method may be used for imaging the stomach and may comprise the step of ingesting a composition to retrain liquids in the body lumen.

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features and advantages thereof, may best be understood by reference to the following detailed description when read with the accompanied drawings in which:
Figure 1 is a block diagram schematically illustrating a system according to an embodiment of the invention;
Figure 2 is a schematic illustration of a sensing device in accordance with an embodiment of the invention;
Figures 3A and 3B are schematic illustrations of an imaging devices being moved through a body lumen in accordance with an embodiment of the invention;
Figure 4 is a schematic illustration of an imaging device according to an embodiment of the invention; and
Figure 5 is a box diagram depicting a method for in vivo imaging making use of an embodiment of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF THE INVENTION

In the following description, various aspects of the present invention will be described. For purposes of explanation, specific configurations and details are set forth in order to provide a thorough understanding of the present invention. However, it will also be appreciated by one skilled in the art that the present invention may be practiced without the specific details presented herein. Furthermore, well known features may be omitted or simplified in order not to obscure the present invention.

A sensing device of the invention, such as an imaging device, may be inserted in vivo. For example, a typically autonomous floatable device may be ingested and moved through the GI tract, typically by peristalsis. When reaching a relatively voluminous liquid filled lumen, such as the stomach, the device, which may float in the volume of liquid, may be moved through the lumen in accordance with the movement of the volume of liquid. Other embodiments may include other suitable devices and methods.

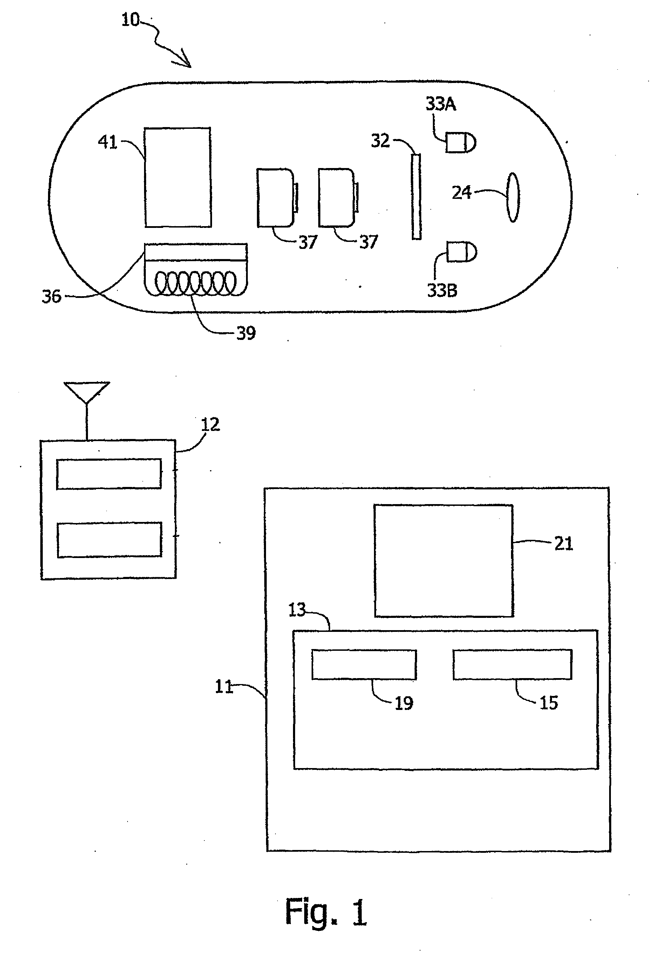

Reference is now made to Fig. 1, which is a schematic illustration of an in vivo sensing device, for example a floatable in vivo imaging device 10, and a data receiver and/or recorder 12 and a workstation 11, according to an embodiment of the present invention. According to one embodiment the imaging device 10 may include an imaging system which may consist of, for example, an imager, an optical system (e.g., lenses or other optical elements) and illumination sources. A data receiver and/or recorder 12 may take other suitable configurations. The data receiver and/or recorder 12 may transfer, for example received information to another computing device, such as a workstation 11 or personal computer, where the data may be further analyzed, stored, and/or displayed to a user.

Device 10 typically may be or may include an autonomous swallowable capsule, but device 10 may have other shapes and need not be swallowable or autonomous. The device may be, for example, similar to embodiments described in U. S. Patent No. 5,604,531 to Iddan et al., and/or WO 01/65995, entitled" A Device And System For In Vivo Imaging", published on 13 September, 2001, both of which are assigned to the common assignee of the present invention. Embodiments of in vivo imaging devices having more than one field of view are described in WO 02/054932 entitled "System and Method for Wide Field Imaging of Body Lumens" published on 18 July, 2002, which is assigned to the common assignee of the present invention. However, the device may be any sort of suitable in-vivo sensor device and may have other configurations.

Embodiments of device 10 are typically autonomous, and are typically self- contained. For example, device 10 may be a capsule or other unit where all the components are substantially contained within a container or shell, and where device 10 does not require any wires or cables to, for example, receive power or transmit information. According to one embodiment of the present invention, device 10 may communicate with data receiver and/or recorder 12 and a workstation 11 to provide display of data, control, or other functions. For example, power may be provided by an internal battery or a wireless receiving system. Other embodiments may have other configurations and capabilities. For example, components may be distributed over multiple sites or units. Control information may be received from an external source.

In one embodiment, all of the components may be sealed within the device body (the body or shell may include more than one piece); for example, an image sensor 32, illumination sources 33A and 33B, power units 37, transmitter 36, antenna 39, and control unit 41, may all be sealed within the device body.

According to one embodiment of the invention the in vivo imaging device may include a sensor. The sensor may be any sensor suitable for sensing in vivo environment parameters, such as pH, temperature, conductivity, shear, pressure and so on. The sensor system can include all the elements necessary for in vivo sensing, as known in the art. In one embodiment the sensor is an image sensor, for example an image sensor such as a CCD or CMOS imager. According to one embodiment of the invention the in vivo imaging device may include one or more illumination source(s) 3A and 3B, such as one or more LEDs (Light Emitting Diode) and/or OLEDs (Organic LED), and an optical system 24 for focusing images onto the image sensor 32. The device may further include a transmitter 36 for transmitting image and other (e.g., non-image) information to a receiving device, and may include other components, such as, for example, a compression module for compressing data. The transmitter 36 is typically an ultra low power radio frequency (RF) transmitter with high bandwidth input, possibly provided in chip scale packaging. The transmitter 36 may also include circuitry and functionality for controlling the device. The transmitter may be, for example, an ASIC, "computer on a chip", microcontroller, etc., or other component. Components such as the image sensor 32, illumination sources 3A and 3B and transmitter 36 may be mounted on a support, which may be, for example, a printed circuit board or plastic board or sheet. The support may be another structure, and components need not be mounted on a separate support. Other suitable components may be used.

According to one embodiment the workstation 11 includes a controller or processor 15, a storage unit 19 and display 21. In other embodiments, each of the various components need not be required; for example, an internal device may transmit or otherwise transfer (e.g., by wire, via radio waves) information directly to a display or processing system 21.

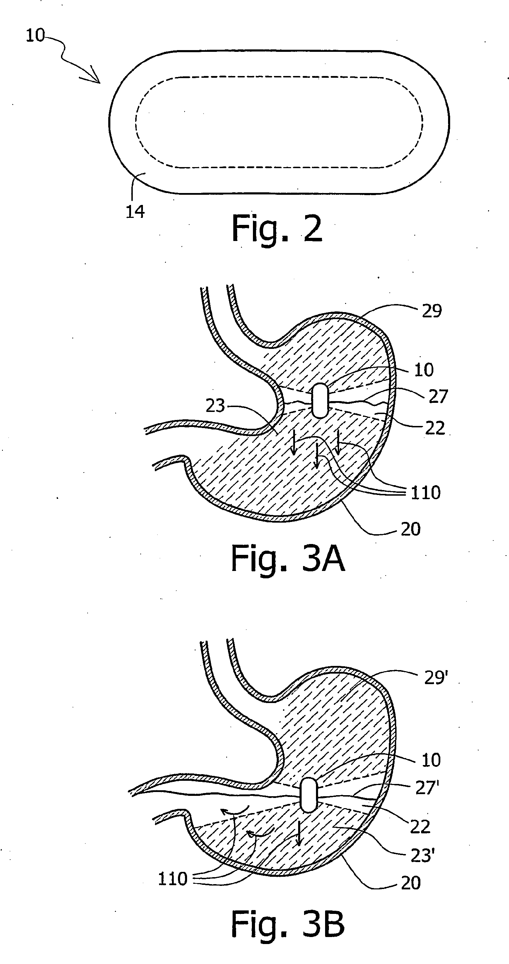

According to embodiments of the invention, a sensing device, for example the floatable in vivo imaging device 10, may have a specific gravity of about 1 or less, or a volume to weight ratio that enables it essentially to float, for example, in a body lumen liquid, for example the liquid typically found in the human stomach, or for example liquid that may be administered to a patient to fill a lumen such as the stomach. According to one embodiment as shown in Fig. 2 the floatable in vivo imaging device 10 may include one or more buoyant bod(ies) 14 or other weight, specific gravity, ballast or mass controlling system. The buoyant body 14, which may be attached to floatable in vivo imaging device 10 or which can optionally house the floatable in vivo imaging device and/or one or more elements of the floatable in vivo imaging device 10, may keep the in vivo imaging device 10 essentially floating in a liquid filled body lumen for example, in a typical human stomach fluid. Embodiments including a floatable imaging device are described, for example, in US application number 2003/0018280 entitled "Floatable in vivo Sensing Device and Method for Use" published on January 23, 2003 which is assigned to the assignee of the present invention. Other suitable specific gravity configurations or floatation systems may be used.

Reference is now made to Figs. 3A and 3B, which schematically illustrate a sensing device, for example the floatable in vivo imaging device 10, being moved through a body lumen in accordance with an embodiment of the invention. According to one embodiment the floatable in vivo imaging device 10 is inserted into a patient's stomach 20, for example, by swallowing. The stomach 20 is filled with a volume of liquid 22, which may be, for example, water, tea, juice, hyperosmolar liquid or any other suitable liquid, which is typically ingested by the patient at about the time of arrival of the imaging device 10 to the stomach 20. According to some embodiments of the present invention, the floatable imaging device 10 will be kept afloat in the liquid 22, enabling the device a multi-directional viewing ability. According to one embodiment the device 10 includes more than one imaging system to enable multi directional viewing and/or imaging. According to some embodiments two imagers, for example situated on opposing sides or ends of the device 10, may enable multi-directional viewing and/or imaging, for example imaging above and below the liquid level. According to one embodiment of the present invention, multi-directional viewing ability may include illumination field and/or a field of view of the area above the liquid level 27, for example area 29. According to other embodiments of the present invention, multi-directional viewing ability may include field of view in the opposite direction, e.g, illumination field and/or a field of view of the area below the liquid level 27, for example area 23, that may be for example filled with a volume of liquid 22. The device may be floatable in the sense that its specific gravity is approximately the same or less than a liquid inserted into or known to be in a body lumen. Other suitable specific gravity levels may be used, and the device need not be floatable in every liquid. Natural action of the GI tract, including the stomach, typically causes liquids to be emptied from lumens, such as the stomach, within a certain time frame. For example, liquid 22 may be naturally emptied from the stomach 20 over a period of 20-30 minutes, while moving in the direction shown, for example, by arrows 110. The floatable imaging device 10, which is carried by the liquid 22, will thus be moved through the stomach in the general direction of the movement of liquid 22. Fig. 3A, for example, schematically illustrates the stomach 20 at a certain time after ingestion of liquid 22, for example, 1-10 minutes after ingestion of liquid 22, whereas Fig.3B schematically illustrates the stomach 20 a certain time after that depicted in Fig. 3A, which may be, for example, 1-50 minutes after ingestion of liquid 22. The liquid level 27' in Fig. 3B is shown to be lower than the liquid level 27 in Fig. 3A. Thus, imaging device 10 may obtain images of field of view 29 and/or field of view 23 at a certain time (as seen, for example, in Fig. 3A), whereas, in a certain time following that, device 10 may obtain images of field of view 29' and/or field of view 23' (as shown, for example, in Fig. 3B). Other fields of view may be obtained. For example device 10 may include more than one imaging system, for example, enabling counter-directional viewing. It should be appreciated that the device 10 may include suitable optical systems for viewing through a liquid as well as for viewing through a substantially non liquid medium. Other suitable specific gravities or floating capabilities may allow for other positions of a device within a liquid volume, and other weight or mass configurations may allow for other imaging directions or orientations within a body lumen.

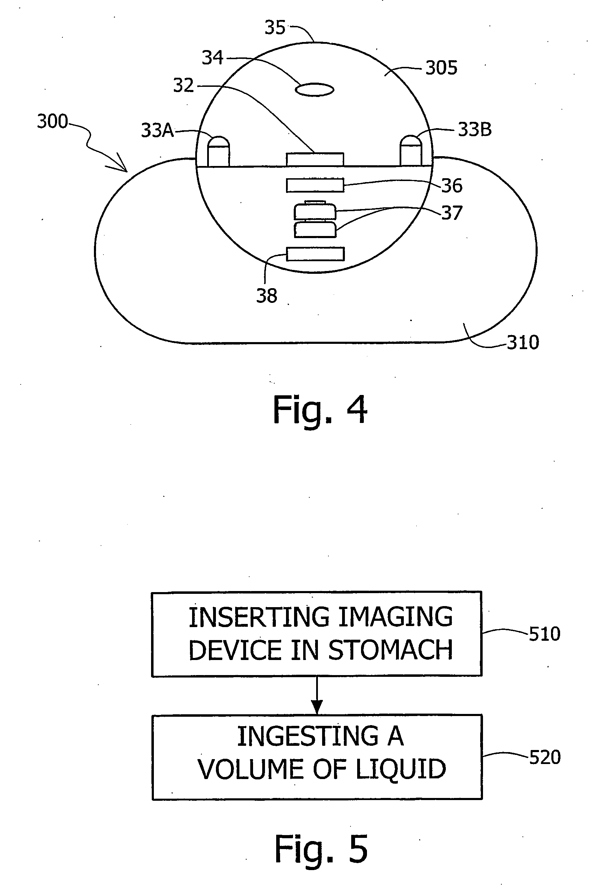

Reference is now made to Fig. 4, which schematically illustrates an imaging device according to one embodiment of the invention. According to one embodiment the buoyant body has a longitudinal axis substantially perpendicular to the optical axis of the imaging system (e.g., the optical axis may substantially coincide with the direction of illumination and imaging). According to one embodiment an imaging device 300 may include an encapsulated imaging system 305 and a buoy 310. According to one embodiment the encapsulated system is substantially spherical and the buoy is typically boat shaped, but the units may have other shapes. One benefit of an embodiment where the buoyant body has a longitudinal axis substantially perpendicular to the optical axis of the imaging system (e.g., the boat shaped device illustrated in Fig. 4) may be its ability to float and/or move through an area in which a liquid level is typically low. For example, a device according to this embodiment may be easily moved through a stomach with a volume of liquid which is less than about 10mm deep (e.g., about 10mm above the lumen floor or wall), whereas other proportioned or shaped floating devices may need a liquid level of about 30mm or above, to be easily moved through the lumen. Other shapes and configurations are possible.

The imaging system 305 may include an image sensor 32, light sources 33A and 33B, and an optical system 34 all positioned behind an optical dome 35. The optical dome 35 may be transparent to enable illumination and/or imaging of a body lumen though it. According to one embodiment the optical dome is made of a glass or plastic such as isoplast™. According to other embodiments other suitable materials may be used.

The imaging system 305 may further include a transmitter 36, typically for transmitting image data to an external receiving unit and a power source, such as batteries 37. The imaging system may be powered externally, thus a battery may not be needed. The imaging system may be attached to or set in one or more buoyant bod(ies). Typically, buoyant body 310 may render device 300 floatable.

Typically, illumination sources 33A and 33B may illuminate a body lumen through optical dome 35 and images of the body lumen may be obtained by image sensor 32. According to one embodiment one or more ballast(s) 38 may be included in the device 300 for allowing one portion of the device 300 or of the imaging system, such as the image sensor 32 and/or illumination sources 33A and 33B, to be usually oriented in a fixed direction, for example, in a counter gravity direction. In alternate embodiments the internal components of a device may be packaged so as to shift the center of gravity (CG) and create ballast in one portion of the device, for example, batteries and electronic components may be packaged at one end of an encapsulated system so as to create ballast at that end. Furthermore, additional equipment may not be needed to, for example, alter the buoyancy or specific gravity of the device or to alter the weight distribution of the device, as such configurations may be done with components already part of the device, such as a gas such as air, CO₂, N₂, etc. occurring within the device, etc. According to some embodiments the buoyant body 31 may serve to keep an imaging system in a fixed orientation. According to embodiments of the invention the buoyant body 310 and/or the ballast 38 may serve to stabilize an imaging system and to ensure floating of the system through a body lumen in a fixed orientation, obtaining relatively smooth imaging. Buoyant body 310 may be filled with air, CO₂, N₂ or another light gas or fluid or any other suitable floatable material so as to render the device 300 floatable.

Reference is now made to Fig. 5, which schematically illustrates a method making use of an embodiment of the invention. Accordingly , in block 510, a floatable imaging device is inserted into a patient's body lumen, for example into the large intestine or stomach, e.g., by swallowing ,the imaging device. In block 520 the patient ingests a volume of liquid, e.g., approximately 100 - 500ml of a hyperosmolar liquid, water, tea, juice etc. According to other embodiments the step of inserting the imaging device may be carried out by placing the device in a specific location of the GI tract, for example, by using an endoscope or another suitable inserting device. According to some embodiments ingesting a volume of liquid may precede the step of inserting an imaging device or a volume of liquid can be can be ingested after the step of inserting an imaging device. According to some embodiments the procedure may be repeated more than once. According to some embodiments the step of ingesting a volume of liquid may be repeated more than once during a procedure whereas each repeat of this step (according to some embodiments after a suitable waiting period, e.g., a minute to tens of minutes between each repeated step) may include ingesting the same or different volumes of liquid and/or different or the same liquids may be used each time. According to some embodiments repeating steps may be utilized for repeated visual scanning or gradual ingestion of a total volume. The total volume may, according to some embodiments, be from approximately 100 ml to a liter or more of liquid.

Time periods, absorption periods, amounts, etc., other than specifically disclosed herein may be used. Other operations or series of operations may be used. According to other embodiments a volume of liquid may be inserted into the patient's stomach by means other than by ingesting, such as by injection.

According to some embodiments a method making use of an embodiment of the invention may include a step of ingesting or otherwise administering to a patient a composition to retain liquids in the body lumen (e.g., in the stomach) and/or to delay passage of liquids from a lumen (e.g., by wakening the peristaltic movements of the stomach). Examples of substances or compositions that may be used in delaying stomach emptying, for example, may include hyperosmolar agents, such as saline or lactulose or high carbohydrate and/or high fat content liquids. According to other embodiments intravenous injection, for example, of glucagon may cause typically short term inhibition of small bowl activity, which may delay stomach emptying. It will be appreciated that a person skilled in the art may adjust the procedure according to specific requirements or patient physiology or physique.

According to some embodiments an in vivo sensing device may be made floatable or have its specific gravity changed after insertion into a patient's body. For example, an imaging device may include a buoy or other floatation or weigh element that may be packaged such that it is not buoyant while in packaging. Release of the buoy from its packaging may lend buoyancy to the sensor system. According to some embodiments of the present invention, the buoy may be released from its packaging at a desired location or point in time, such that the floatable in vivo imaging device may acquire buoyancy according to specific requirements. For example, the floatable in vivo imaging device 10 according to an embodiment of the invention may be ingested and moved by peristalsis through the esophagus while its buoy is packaged. When the device enters the stomach the buoy is released from its packaging (for example, by using a pH sensitive mechanism, as known in the art, or other suitable mechanism) and the device can then float in a bulk of liquid in the stomach and be carried by the bulk of liquid to all areas of the stomach and may be carried down through the whole stomach, enabling, for example, imaging of substantially most of the stomach walls. The mechanisms by which the buoy is released from its packaging can be externally controlled or automatically controlled, for example, as described in embodiments described in the above mentioned US application number 2003/0018280.

It will be appreciated by persons skilled in the art that the present invention is not limited by what has been particularly shown and described herein above. Rather the scope of the invention is defined by the claims, which follow.

## Claims

1. A swallowable in-vivo capsule (10) for in vivo imaging, the capsule (10) comprising:
a buoyant body (14);
a first imaging system (32, 33A); and
a second imaging system (32, 33B);
**characterized in that** the buoyant body (14) is to provide buoyancy to the capsule (10), and internal components of the capsule (10) are arranged to adjust a centre of gravity of the capsule (10) such that, when the capsule is floating at a surface of a liquid, the capsule assumes an orientation such that the first imaging system (32, 33A) images above the liquid surface and the second imaging system (32, 33B) images below the liquid surface.

2. The capsule (10) according to claim 1, wherein the first imaging system (32, 33A) and the second imaging system (32, 33B) are situated on opposing ends of the buoyant body (14).

3. The capsule (10) according to claim 1 or 2, wherein the capsule (10) is an autonomous swallowable capsule (10).

4. The capsule (10) according to any preceding claim, having a specific gravity of about 1.

5. The capsule (10) according to any preceding claim, comprising a transmitter (36).

6. The capsule (10) according to any preceding claim, wherein the buoyant body (14) houses the capsule (10).

7. The capsule (10) according to any preceding claim, wherein the buoyant body (14) has a longitudinal axis substantially perpendicular to an optical axis of the first imaging system (32, 33A) and substantially perpendicular to an optical axis of the second imaging system (32, 33B).

8. The capsule (10) according to any preceding claim, wherein the buoyant body (14) is configured to cause the capsule (10) to float at a surface of a liquid in a body lumen in a substantially fixed orientation, with the first imaging system (32, 33A) imaging above a liquid level and the second imaging system (32, 33B) imaging below the liquid level when the capsule (10) is floating in the liquid.

9. The capsule (10) according to any preceding claim, further comprising ballast (38) to keep the imaging systems (32, 33A, 33B) in a substantially fixed orientation when the capsule (10) is floating in liquid in a body lumen.

10. The capsule (10) according to claim 9, wherein the ballast (38) is provided by packaging internal components of the capsule (10) in one portion of the capsule (10).

11. The capsule (10) according to any preceding claim, wherein the buoyant body (14) includes a floatable fluid.

12. The capsule (10) according to any preceding claim, wherein the capsule (10) has first and second ends, and wherein the buoyant body (14) is configured to cause the capsule (10), when floating at a surface of a liquid in a body lumen, to float with the first end above a liquid level and the second end below the liquid level.

## Patentansprüche

1. Schluckbare In-vivo-Kapsel (10) für eine In-vivo-Bildgebung, wobei die Kapsel folgendes umfasst:
einen Auftriebskörper (14);
ein erstes bildgebendes System (32, 33A); und
ein zweites bildgebendes System (32, 33B);
**dadurch gekennzeichnet, dass** der Auftriebskörper (14) dazu dient, der Kapsel (10) Auftrieb zu verleihen, und wobei die inneren Komponenten der Kapsel (10) so angeordnet sind, dass sie den Schwerpunkt der Kapsel (10) so anpassen, dass wenn die Kapsel auf einer Oberfläche einer Flüssigkeit schwimmt, die Kapsel eine Ausrichtung annimmt, so dass das erste bildgebende System (32, 33A) oberhalb der Flüssigkeitsoberfläche bildgebend ist, und wobei das zweite bildgebende System (32, 33B) unterhalb der Flüssigkeitsoberfläche bildgebend ist.

2. Kapsel (10) nach Anspruch 1, wobei das erste bildgebende System (32, 33A) und das zweite bildgebende System (32, 33B) an entgegengesetzten Enden des Auftriebskörpers (14) angeordnet sind.

3. Kapsel (10) nach Anspruch 1 oder 2, wobei es sich bei der Kapsel (10) um eine autonome, schluckbare Kapsel (10) handelt.

4. Kapsel (10) nach einem der vorstehenden Ansprüche, mit einer spezifischen Dichte von etwa 1.

5. Kapsel (10) nach einem der vorstehenden Ansprüche, mit einem Sender (36).

6. Kapsel (10) nach einem der vorstehenden Ansprüche, wobei sich die Kapsel (10) in dem Auftriebskörper (14) befindet.

7. Kapsel (10) nach einem der vorstehenden Ansprüche, wobei der Auftriebskörper (14) eine Längsachse aufweist, die im Wesentlichen senkrecht ist zu einer optischen Achse des ersten bildgebendes Systems (32, 33A), und wobei sie ferner im Wesentlichen senkrecht ist zu einer optischen Achse des zweiten bildgebendes Systems (32, 33B).

8. Kapsel (10) nach einem der vorstehenden Ansprüche, wobei der Auftriebskörper (14) so konfiguriert ist, dass er es bewirkt, dass die Kapsel (10) auf einer Oberfläche einer Flüssigkeit in einem Körperlumen in einer im Wesentlichen festen Ausrichtung schwimmt, wobei das erste bildgebende System (32, 33A) oberhalb einer Flüssigkeitsebene bildgebend ist, und wobei das zweite bildgebende System (32, 33B) unterhalb der Flüssigkeitsebene bildgebend ist, wenn die Kapsel (10) in der Flüssigkeit schwimmt.

9. Kapsel (10) nach einem der vorstehenden Ansprüche, wobei diese ferner Ballast (38) umfasst, um die bildgebenden Systeme (32, 33A, 33B) in einer im Wesentlichen flachen Ausrichtung zu halten, wenn die Kapsel (10) in einer Flüssigkeit in einem Körperlumen schwimmt.

10. Kapsel (10) nach Anspruch 9, wobei der Ballast (38) bereitgestellt wird durch die Anordnung von inneren Komponenten der Kapsel (10) in einem Teilstück der Kapsel (10).

11. Kapsel (10) nach einem der vorstehenden Ansprüche, wobei der Auftriebskörper (14) ein schwimmfähiges Fluid aufweist.

12. Kapsel (10) nach einem der vorstehenden Ansprüche, wobei die Kapsel (10) erste und zweite Enden aufweist, und wobei der Auftriebskörper (14) so konfiguriert ist, dass er es bewirkt, wenn die Kpasel (10) auf einer Oberfläche einer Flüssigkeit in einem Körperlumen schwimmt, dass sie mit dem ersten Ende oberhalb einer Flüssigkeitsebene und mit dem zweiten Ende unterhalb der Flüssigkeitsebene schwimmt.

## Revendications

1. Capsule in vivo à avaler (10) pour une imagerie in vivo, la capsule (10) comprenant :
un corps flottant (14) ;
un premier système d'imagerie (32, 33A) ; et
un second système d'imagerie (32, 33B) ;
**caractérisée en ce que** le corps flottant (14) assure la flottabilité de la capsule (10), et les composants internes de la capsule (10) sont disposés pour ajuster un centre de gravité de la capsule (10) de telle sorte que, lorsque la capsule flotte à la surface d'un liquide, la capsule prend une orientation telle que le premier système d'imagerie (32, 33A) image au-dessus de la surface du liquide et le second système d'imagerie (32, 33B) image en dessous de la surface du liquide.

2. Capsule (10) selon la revendication 1, dans lequel le premier système d'imagerie (32, 33A) et le second système d'imagerie (32, 33B) sont situés sur des extrémités opposées du corps flottant (14).

3. Capsule (10) selon la revendication 1 ou 2, dans lequel la capsule (10) est une capsule à avaler autonome (10).

4. Capsule (10) selon l'une quelconque des revendications précédentes, ayant une densité spécifique d'environ 1.

5. Capsule (10) selon l'une quelconque des revendications précédentes, comprenant un émetteur (36).

6. Capsule (10) selon l'une quelconque des revendications précédentes, dans laquelle le corps flottant (14) contient la capsule (10).

7. Capsule (10) selon l'une quelconque des revendications précédentes, dans laquelle le corps flottant (14) a un axe longitudinal sensiblement perpendiculaire à un axe optique du premier système d'imagerie (32, 33A) et sensiblement perpendiculaire à un axe optique du second système d'imagerie (32, 33B).

8. Capsule (10) selon l'une quelconque des revendications précédentes, dans laquelle le corps flottant (14) est configuré pour amener la capsule (10) à flotter à la surface d'un liquide dans une lumière corporelle dans une orientation sensiblement fixe, le premier système d'imagerie (32, 33A) imageant au-dessus d'un niveau de liquide et le second système d'imagerie (32, 33B) imageant en dessous du niveau du liquide lorsque la capsule (10) flotte dans le liquide.

9. Capsule (10) selon l'une quelconque des revendications précédentes, comprenant en outre un ballast (38) pour maintenir les systèmes d'imagerie (32, 33A, 33B) dans une orientation sensiblement fixe lorsque la capsule (10) flotte dans du liquide dans une lumière corporelle.

10. Capsule (10) selon la revendication 9, dans laquelle le ballast (38) est fourni en emballant des composants internes de la capsule (10) dans une partie de la capsule (10).

11. Capsule (10) selon l'une quelconque des revendications précédentes, dans laquelle le corps flottant (14) contient un fluide flottable.

12. Capsule (10) selon l'une quelconque des revendications précédentes, dans laquelle la capsule (10) a des première et seconde extrémités, et dans laquelle le corps flottant (14) est configuré pour amener la capsule (10), lorsqu'elle flotte à la surface d'un liquide dans une lumière corporelle, à flotter avec la première extrémité au-dessus d'un niveau de liquide et la seconde extrémité en dessous du niveau du liquide.
